# EUROPÄISCHE PATENTANMELDUNG

(11) **EP 0 875 193 A2**
(43) Veröffentlichungstag der Anmeldung: **04.11.1998**
(21) Anmeldenummer: 98250142.1
(22) Anmeldetag: 23.04.1998
(51) Int. Cl.: A47K 11/06, A61F 5/455

(54) **Urin- und Exkrementeableiter und dessen Verwendung**

(30) Priorität: 23.04.1997 DE 19717853
(71) Anmelder: Römpke, Heinz, D-23669 Timmendorfer Strand (DE)
(72) Erfinder: Römpke, Heinz, D-23669 Timmendorfer Strand (DE)
(74) Vertreter: Scholz, Hartmut, Dipl.-Ing. Patentanwalt

(57) **Zusammenfassung**

Vorrichtung (10) zur Ableitung von Urin und Exkremente bei einem seine Notdurft verrichtenden Menschen, bestehend aus einem flachen Bahnmaterial mit einem eine Einlaßöffnung (12) aufweisenden oberen Bereich (11), einem eine Auslaßöffnung (14) aufweisenden unteren Bereich (11a) und einer Oberfläche (21), auf der ein das Öffnen der Einlaßöffnung (12) vereinfachendes Haftmittel (19) aufgebracht ist. Weiteren dient die Vorrichtung (20) zum Ableiten von Exkrementen eines einen unnatürlichen Darmausgang oder Harnausgang aufweisenden Lebewesen, insbesondere eines Menschen.

## Beschreibung

Die Erfindung betrifft eine Vorrichtung zum Ableiten von Urin und/oder Exkrementen bei einem seine Notdurft verrichtenden Menschen, bestehend aus einem blattförmigen, flachen Bahnmaterial, das zu einem Ableitschlauch zusammengelegt ist, mit einem eine Einlaßöffnung aufweisenden oberen Bereich und einem eine Auslaßöffnung aufweisenden unteren Bereich.

Diese Erfindung dient Menschen, die aus den verschiedensten Gründen im Stehen urinieren oder beispielsweise einen Stomabeutel diskret entleeren wollen. Aufgrund der Fallhöhe und dem schwer zu beeinflussenden Streuwinkel kommt es oft zu Verspritzungen, die sich in Form von kleinen Tröpfchen außerhalb der Toilettenschüssel oder auf der Kleidungen absetzen können.

Aus der DE 43 43 789 C2 ist ein Urinableiter bekannt, der aus einem beidseitig offenen, flexiblen Schlauch besteht, der aus einem sich zeitlich verzögert in Wasser zumindest teilweise auflösenden Material gefertigt ist. Der schlauchförmige Urinableiter weist eine trichterförmige Einlaßöffnung auf und ist im oberen Bereich mit Haltemitteln und einem eine Auslaßöffnung aufweisenden unteren Bereich versehen.

Aus der DE 44 43 182 C2 ist eine Vorrichtung zur Ableitung von Urin bei einer seine Notduft verrichtenden, insbesondere männlichen Person bekannt. Diese Vorrichtung besteht aus einem flexiblen Schlauch, der aus einem Flachmaterial aus doppeltgelegten Einzelblättern, beispielsweise aus Papier, gefertigt und mit einem Haltebereich, einem Einlaufbereich und einem Auslaufbereich versehen ist. Die Einzelblätter sind in einer Vielzahl quasi endlos aneinandergereiht und an ihrer gemeinsamen Vorderkante in einer längsverlaufenden Hohlkehle einstückig miteinander verbunden.

Aus der US 4,608,046 ist eine Vorrichtung zum Ableiten von Urin bei Frauen bekannt, bestehend aus einer an die Labia zu führenden Röhre. Diese Röhre ist flexibel und paßt sich somit dem entsprechend Anwendungsfall an.

Bei allen beschriebenen Vorrichtungen ist ein Öffnen der Einlaßöffnung problematisch. Durch das flache Zusammenlegen ist zwar ein platzsparender, kostengünstiger Transport und eine einfachere Lagerung möglich, umständlich ist jedoch das Auseinanderfalten und Öffnen der Einlaßöffnung, da dem Benutzer dafür in der Regel nur eine Hand zur Verfügung steht.

Aufgabe der vorliegenden Erfindung ist es, einen die Fallhöhe reduzierenden und Körperausscheidungen kanalisierenden Ableiter zu schaffen, der einfach und sauber zu handhaben ist und bei dem die Einlaßöffnung schnell und sicher geöffnet werden kann.

Gelöst wird die Aufgabe dadurch, daß dem Einlaßbereich ein Haltemittel zugeordnet und im Bereich der Einlaßöffnung eine Haftmittelbeschichtung auf die Oberfläche des Bahnmaterials aufgetragen ist.

Das Haftmittel ist im oberen Bereich angrenzend in der Einlaßöffnung angeordnet. Das Haftmittel ist horizontal umlaufend innerhalb eines Haftmittelbereichs auf der Oberfläche aufgebracht, wodurch der Daumen und der Zeigefinger genügend Kraft auf die flach zusammengefaltete Vorrichtung ausüben können, um eine Öffnung im Bereich des Einlaß zu formen.

Lebewesen, insbesondere Menschen, deren Harnleiter bzw. Darm aufgrund einer Operation in einer unnatürlichen Weise aus dem Körper geführt wird, haben oftmals Probleme, ihre Exkremente kontrolliert und diskret zu entsorgen. Diese Menschen haben beispielsweise durch eine Krebserkrankung und anschließender Operation Teile ihres Darms verloren. Der Darm- bzw. Harnausgang wird künstlich aus dem Körper geführt und mit einem Entleerungsventil versehen, an das bei Bedarf ein Katheter angeschlossen wird. Aufgrund dieser Anordnung ist eine dosierte Abgabe der Exkremente nur schwer möglich.

Gelöst wird dieses Problem durch eine Vorrichtung, bestehend aus einem sich in Wasser zeitverzögert zumindest teilweise auflösenden, flachen Bahnmaterial, das einen flexiblen Schlauch bildet, mit einem eine Einlaßöffnung aufweisenden oberen Bereich und einem eine Auslaßöffnung aufweisenden unteren Bereich, und die Vorrichtung zum Ableiten von Exkrementen eines einen unnatürlichen Darmausgang oder Harnausgang aufweisenden Lebewesens dient.

Weitere vorteilhafte Maßnahmen sind in den Unteransprüchen beschrieben. Die Erfindung ist in der beiliegenden Zeichnung dargestellt und wird nachfolgend näher beschrieben; es zeigt:
- **Figur 1**: die Seitenansicht eines erfindungsgemäßen Urinableiters, mit einem oberen Bereich, in dem eine Einlaßöffnung angeordnet ist, und einem unteren Bereich, der eine Auslaßöffnung aufweist, mit einem Einschnitt und einem Haftmittelbereich, in dem ein Haftmittel aufgetragen ist;
- **Figur 2**: die Draufsicht des in Figur 1 beschriebenen Urinableiters;
- **Figur 3**: zeigt die Seitenansicht des in Figur 1 beschriebenen Urinableiters, mit ausschließlich einer vorderen Verbindungsnaht und einem oberhalb des Einschnitts angeordneten Haftmittelbereich;
- **Figur 4**: die Draufsicht auf eine Vorrichtung zum Ableiten von Exkrementen, mit aufklappbarer Einlaßöffnung;
- **Figur 5**: die Seitenansicht eines Exkrementeableiters nach der Figur 4, mit aufgeklappter Einlaßöffnung und darin eingeführtem, zu entleerenden Stomabeutel;
- **Figur 6**: die Draufsicht auf eine Vorrichtung zum Ableiten von Urin, mit asymmetrisch angeordnetem Ableitschlauch und aufgeklappter Einlaßöffnung;
- **Figur 7**: die Draufsicht auf eine Vorrichtung zum Ableiten von Urin, mit asymmetrisch angeordnetem Ableitschlauch und aufgeklappter Einlaßöffnung nach der Figur 6, durch einen separaten Haltetrichter gezogen;
- **Figur 8**: die Draufsicht auf einen separaten Haltetrichter nach der Figur 7, zur Aufnahme einer Vorrichtung zum Ableiten von Urin, mit asymmetrisch angeordneten Ableitschlauch;
- **Figur 9**: die Draufsicht auf einen separaten Haltetrichter nach den Figuren 7 und 8, zur Aufnahme einer Vorrichtung zum Ableiten von Urin, mit asymmetrisch angeordneten Ableitschlauch, mit unterschiedlichen Verbindungsmitteln zum Schließen seiner Hinterkante.

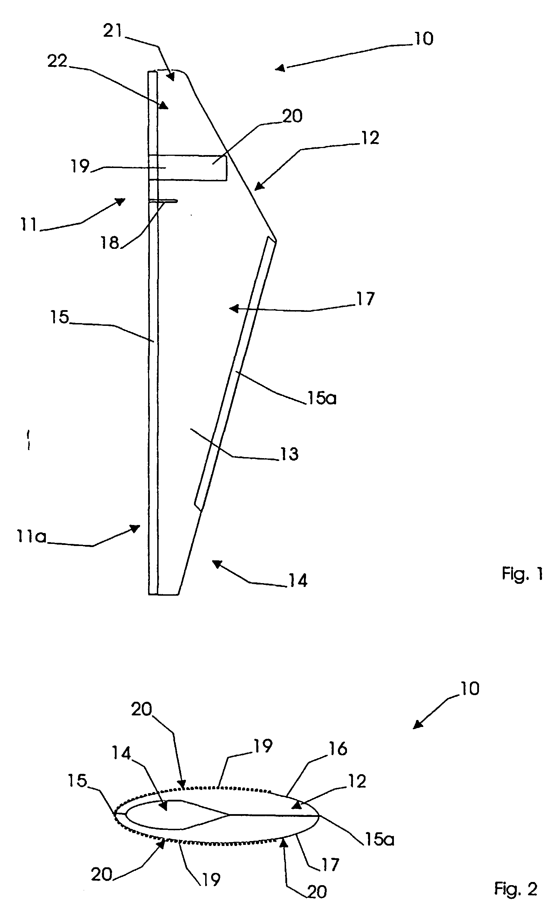

Der in der Figur 1 dargestellte Urinableiter 10 besteht aus konisch nach unten verlaufenden Blättern 16 und 17. Zur Bildung eines Ableitschlauches 13 liegen die Blätter 16 und 17 flächendeckend übereinander und sind an ihren Kanten bereichsweise durch Verbindungsnähte 15 und 15a miteinander verbunden.

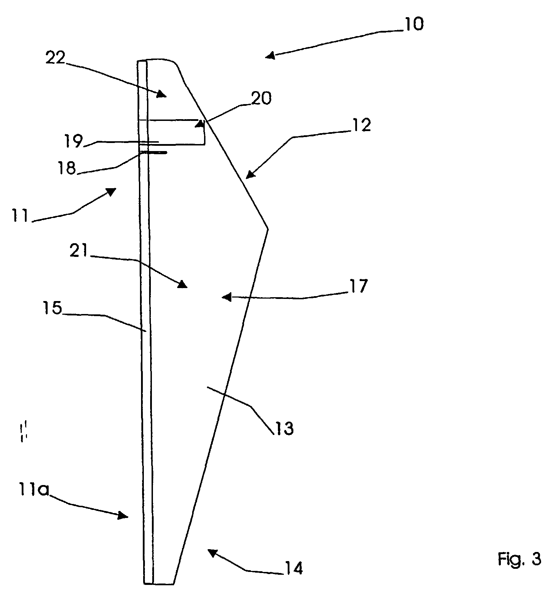

Figur 3 zeigt eine Ausführungsform des Urinableiters 10, bei der die Blätter 16 und 17 nur durch eine vordere Verbindungsnaht 15 miteinander verbunden sind. Diese Ausführungsform ermöglicht eine einfache, kostengünstige Herstellung, da eine Verbindungsnaht weniger verklebt werden muß.

In seinem oberen Bereich 11 weist der Urinableiter 10 eine Einlaßöffnung 12 auf. Die Einlaßöffnung 12 ist schräg nach oben verlaufend zur vertikal verlaufenden Verbindungsnaht 15 angeordnet, wodurch eine vom Körper abweisende Ausrichtung des Urinableiters 10 nach dem Einführen des Penis erreicht wird.

Im unteren Bereich 11a weist der Urinableiter 10 eine Auslaßöffnung 14 auf, aus der der Urin in ein entsprechendes Auffangmittel, beispielsweise eine - nicht dargestellte - Toilettenschüssel geleitete werden kann.

Im oberen Bereich 11 und im Bereich der Einlaßöffnung 12 ist ein horizontal verlaufender Haftmittelbereich 20 vorgesehen, der mit einem Haftmittel 19 versehen ist. Wie die Figur 2 zeigt, verläuft der Haftmittelbereich 20 umlaufende auf der Oberfläche 21 des Urinableiters 10. Der Haftmittelbereich 20 ist vorzugsweise streifenförmig ausgebildet. Die Haftkraft des Haftmittels 19 ist recht gering und erhöht lediglich die Oberflächenreibung innerhalb des Haftmittelbereichs 20 relativ zur restlichen Oberfläche 21.

Das Haftmittel 19 ist vorzugsweise eine innerhalb des Haftmittelbereichs 20 aufgetragene Schicht. Für den Endverbraucher sind die Urinableiter 10 in einer größeren Stückzahl auf einer Rolle abgelegt, wie z.B. Toilettenpapier, oder in einem Kasten verpackt.

Um ein Verkleben der einzelnen Urinableiter in ihrer Verpackung zu verhindern, sollte das Haftmittel, nach dem es aufgetragen und bevor es verpackt wurde, eine Trockenzeit von weniger als einer Minute aufweisen. Ein mögliches Haftmittel 19 mit den beschriebenen Eigenschaften ist eine Gummierung oder eine Kaschierung mit einer Melasse.

Derartige Kaschierungen bewirken auch eine zeitlich begrenzte Feuchtigkeitsresitenz und lösen sich dann rückstandsfrei auf. Eine einfache Entsorgung durch Wegspülen mit einer üblichen Wasserspülung in einer Toilette ist damit ermöglicht.

Eine anderer Möglichkeit das Öffnen der Einlaßöffnung zu erleichtern besteht darin, die Oberfläche 21 der Einzelblätter 16 und 17 im Haftmittelbereich 19 mechanisch oder chemisch aufzurauhen. Dieses kann durch ein entsprechendes Anschleifen oder durch Auftragen einer Flüssigkeit geschehen, wodurch die Oberflächenstruktur griffiger wird.

Um ein Aufsteigen von Feuchtigkeit, insbesondere des Urins innerhalb der beim Gebrauch feucht werdenden Blätter 16 und 17 zu vermeiden, ist ein im wesentlichen waagerecht verlaufender Einschnitt 18 vorgesehen. Der Einschnitt 18 grenzt einen sich von der Oberkante des Urinableiters 10 sich nach unten erstreckenden Haltebereich 22 ab.

Der im wesentlichen waagerecht verlaufende Einschnitt 18 unterbricht die innerhalb des Materials der Blätter 16 und 17 vorhandenen Kapillare. Somit wird der Haltebereich 22 von aufsteigender Feuchtigkeit freigehalten, und eine Benetzung der Finger des Benutzers wird vermieden.

Der Haftmittelbereich 20 und der Haftebereich 22 sind oberhalb des Einschnitts 18 angeordnet, wodurch das Haftmittel 19 nicht nur zum Öffnen der Einlaßöffnung 12 sondern auch zum sicheren Halten des Urinableiters 10 dienen kann.

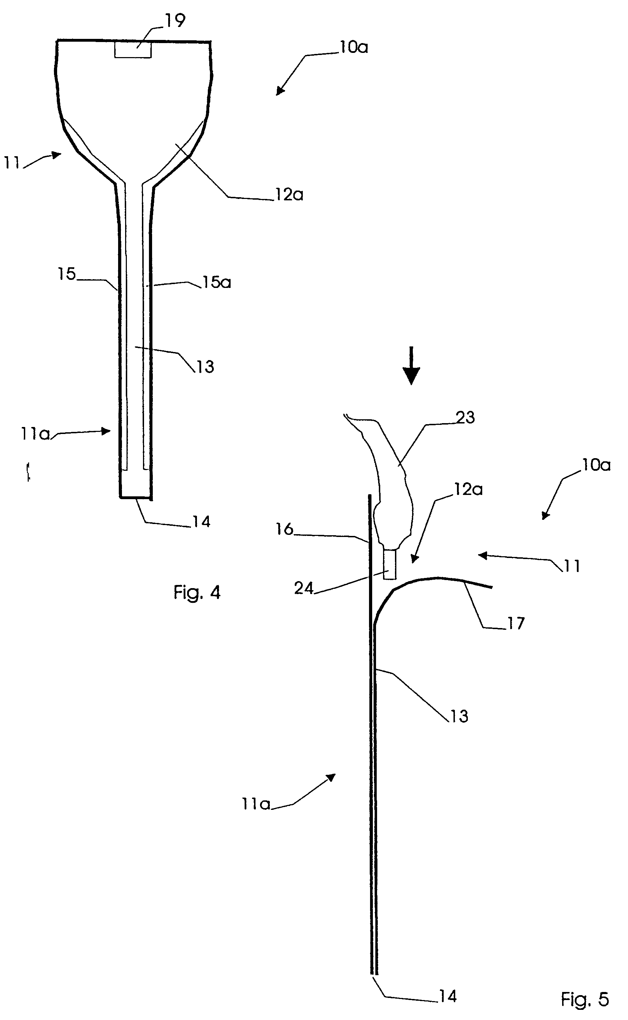

In den Figuren 4 und 5 ist ein Ableiter 10a dargestellt, mit dem in einem Stomabeutel 23 angesammelte Exkremente entsorgt werden können. Der obere Bereich 11 dieses Exkrenteableiters 10a ist dazu mit einer trichterförmig erweiterten Klappöffung 12a versehen.

Zu seiner Entleerung wird, wie die Figur 5 zeigt, das vordere Blatt 17 vom - nicht dargestellten - Körper des Benutzers weggeklappt und das hintere Blatt 16 unter den Stomabeutel 23 geschoben werden. Die Beutelöffung 24 des Stomabeutels 23 wird eingeführt und das vordere Blatt 17 wieder zurückgeklappt. Das hintere Blatt 16 dient dabei zugleich als Haltemittel für den Exkrementeableiter 10a. Zum Ausstreifen des Stomabeutels 23 wird die Beutelöffnung 24 direkt über die Einlaßöffnung des Ableitschlauches 13 gelegt und der Beutelinhalt kann spritzerfrei und sauber entsorgt werden.

Um die Ableitung und den Austritt von festen oder pastösen Bestandteilen aus dem Stomabeutel 23 zu erleichtern, können der untere Bereich 11a des Ableitschlauch 13 und die untere Auslaßöffnung 14 erweitert sein.

Um eine vorzeitige Durchfeuchtung der Blätter 16 und 17 zu vermeiden, ist deren Innenoberfläche kaschiert, vorzugsweise mit Melasse. Melasse ist ein natürliches Rückstandsprodukt aus der Zuckerrübenverarbeitung, das sich zeitlich verzögert in Wasser auflöst, ohne feste Rückstände zu hinterlassen. Diese Kaschierung bewirkt somit eine zeitliche Verzögerung bei der Durchfeuchtung der Blätter 16 und 17 und löst sich nach Gebrauch umweltfreundlich mit zeitlicher Verzögerung quasi rückstandsfrei auf.

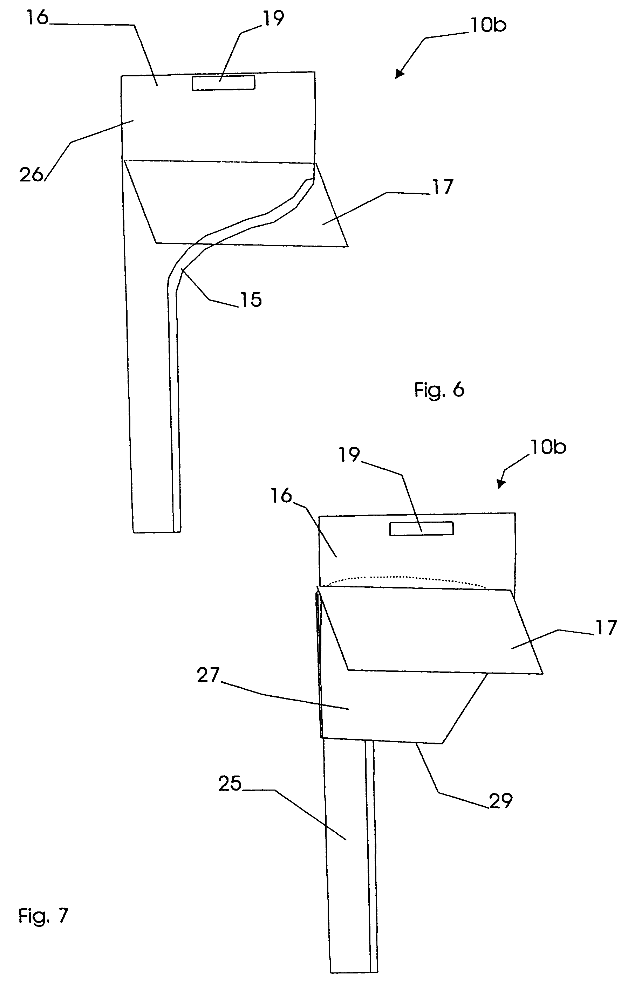

Häufig besteht auch für Damen die Notwendigkeit, ihre Notdurft stehend zu verrichten, insbesondere bei unklaren hygienischen Verhältnissen im Bereich fremder Toiletten. Dazu ist ein Urinableiter 10b vorgesehen, wie er in den Figuren 6 und 7 dargestellt ist.

Dieser Urinableiter 10b weist einen asymmetrischen Trichterbereich 26 auf, der einstückig in einen asymmetrischen Ableitschlauch 25 übergeht. Der asymmetrische Trichterbereich 26 besteht aus zwei gegeneinander geklappten Blättern 16 und 17, die zur besseren Trennung an ihrem oberen Rand ebenfalls mit einer Haftmittelbeschichtung 19 versehen sind. Durch die Haftmittelbeschichtung 19 wird das Aufklappen der Blätter 16 und 17 erleichtert.

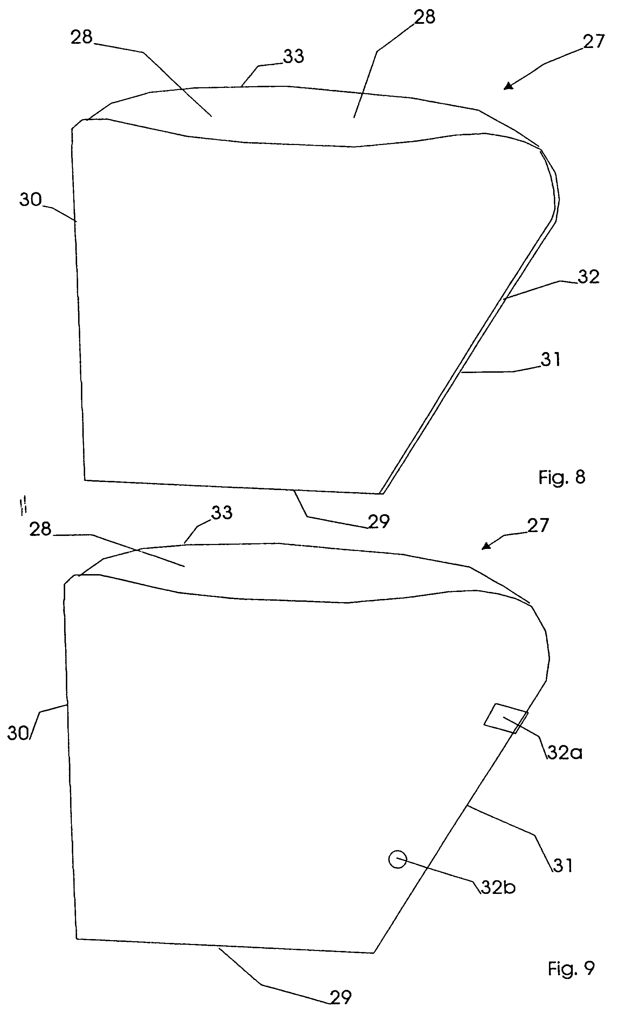

Wie die Figur 7 zeigt, kann der asymmetrische Urinableiter 10b in einen Haltetrichter 27 eingeführt und zum Verrichten der Notdurft gehalten werden. Wie die Figuren 8 und 9 zeigen, ist der Haltetrichter 27 mit einer oberen, größeren Trichteröffnung 28 und einer kleineren, unteren Trichteröffnung 29 versehen.

Der asymmetrische Urinableiter 10b kann mit seinem asymmetrischen Ableitschlauch 25 durch die große obere Trichteröffnung 28 eingesteckt und nach unten durch die kleinere untere Trichteröffnung 29 des Haltetrichters 27 herausgezogen werden. Danach können die Blätter 16 und 17 gegen die Außenoberflächen des Haltetrichters 27 umgeklappt werden.

Der Haltetrichter 27 besteht aus einem flexiblen, etwas härteren Material als es der Urinableiter 10b ist. An seiner Vorderkante 30 ist der Haltetrichter 27 einstückig umgebogen ausgebildet. Die beiden in der Vorderkante 30 umgebogenen Seitenteile des Haltetrichters 27 sind an der Hinterkante 31 zusammengeführt und durch Verbindungsmittel, beispielsweise einer Verbindungsnaht 32 miteinander verbunden.

Zur Benutzung können die Vorderkante 30 und die Hinterkante 31 gegeneinander gedrückt werden. Dadurch wird die obere Trichteröffnung 28 aufgeweitet und der Haltetrichter 27 geöffnet. Der obere Rand 33 des Haltetrichters 27 ist konkav geschwungen und in etwa der Anatomie einer Benutzerin angepaßt.

Wie die Figur 9 zeigt, kann die Hinterkante 31 des Haltetrichters 27 auch mit anderen Verbindungsmitteln geschlossen sein. So sind beispielsweise punktuelle Klebestellen oder Klettungen 32a oder Druckknopfverbindungen 32b vorgesehen.

Da der Haltetrichter 27 lediglich zur Aufnahme eines Urinableiters 10 oder 10b oder eines Exkrementeableiters 10a dient, kommt er mit Urin oder den Exkrementen nicht in Verbindung und wird auch nicht beschmutzt. Nach dem Gebrauch kann der jeweilige Ableiter 10, 10a oder 10b nach unten durch den Haltetrichter 27 durchgezogen und entsorgt werden.

Auch der asymmetrische Urinableiter 10b ist innen kaschiert, beispielsweise mit Melasse. Er löst sich zeitlich verzögert in Wasser auf, ohne feste Rückstände zu hinterlassen. Diese Kaschierung bewirkt somit auch hier eine zeitliche Verzögerung bei der Durchfeuchtung der Blätter 16 und 17. Der asymmetrische Urinableiter 10b löst sich nach Gebrauch umweltfreundlich mit zeitlicher Verzögerung und quasi rückstandsfrei auf.

### Bezugszeichen

- 10, 10b: Urinableiter
- 10a: Exkrementeableiter
- 11: oberer Bereich
- 11a: unterer Bereich
- 12: Einlaßöffnung
- 12a: Klappöffnung
- 13: Ableitschlauch
- 14: Auslaßöffnung
- 15, 15a: Verbindungsnaht
- 16, 17: Blatt
- 18: Einschnitt
- 19: Haftmittel
- 20: Haftmittelbereich
- 21: Oberfläche
- 22: Haltebereich
- 23: Stomabeutel
- 24: Beutelöffnung
- 25: Ableitschlauch, asymmetrisch
- 26: Trichterbereich, asymmetrisch
- 27: Haltetrichter
- 28: Trichteröffnung, oben
- 29: Trichteröffnung, unten
- 30: Vorderkante
- 31: Hinterkante
- 32, 32a, 32b: Verbindungsmittel
- 33: konkaver Rand

## Patentansprüche

1. Vorrichtung zum Ableiten von Urin und/oder Exkrementen bei einem seine Notdurft verrichtenden Menschen, bestehend aus einem blattförmigen, flachen Bahnmaterial, das zu einem Ableitschlauch zusammengelegt ist, mit einem eine Einlaßöffnung aufweisenden oberen Bereich und einem eine Auslaßöffnung aufweisenden unteren Bereich, dadurch gekennzeichnet, daß dem Einlaßbereich (12, 12a, 26) ein Haltemittel (16, 22, 27) zugeordnet und im Einlaßbereich (12, 12a, 26) eine Haftmittelbeschichtung (19) auf die Oberfläche (21) des Bahnmaterials (16, 17) aufgetragen ist.

2. Vorrichtung nach Anspruch 1, dadurch gekennzeichnet, daß das Haftmittel (19) streifenförmig in einem Haftmittelbereich (20) auf der Oberfläche (21) im Bereich der Einlaßöffnung (12) angeordnet ist, wobei die Oberflächenreibung innerhalb des Haftmittelbereichs (20) relativ zur restlichen Oberfläche (21) vergrößert.

3. Vorrichtung nach den Ansprüchen 1 und 2, dadurch gekennzeichnet, daß die Blätter (16, 17) flächendeckend übereinanderliegen und an ihren äußeren Kanten durch Verbindungsnähte (15, 15a) zumindest teilweise oder nur an ihren vorderen Kanten durch eine Verbindungsnaht (15) miteinander verbunden sind.

4. Vorrichtung nach den Ansprüchen 1 bis 3, dadurch gekennzeichnet, daS der Haftmittelbereich (20) oberhalb eines Einschnitts (18) angeordnet ist, wobei der Einschnitt (18) die Blätter (16, 17) im oberen Bereich (11) partiell durchtrennt.

5. Vorrichtung bestehend aus einem sich in Wasser zeitlich verzögert zumindest teilweise auflösenden, flachen Bahnmaterial, das einen flexiblen Schlauch bildet, mit einem eine Einlaßöffnung aufweisenden oberen Bereich und einem eine Auslaßöffnung aufweisenden unteren Bereich, und die Vorrichtung zum Ableiten von Exkrementen eines einen unnatürlichen Darmausgang oder Harnausgang aufweisenden Lebewesens dient.

6. Vorrichtung nach den Ansprüchen 1 bis 5, dadurch gekennzeichnet, daß der Urinableiter (10a) mit einem asymmetrischen Trichterbereich (26) versehen ist und der asymmetrischen Trichterbereich (26) in einen asymmetrisch angeformten Ableitschlauch (25) übergeht.

7. Vorrichtung nach den Ansprüchen 1 und 6, dadurch gekennzeichnet, daß der Urinableiter (10a) mit seinem asymmetrischen Trichterbereich (26) mit einem Haltetrichter (27) in Wirkverbindung bringbar ist.

8. Vorrichtung nach Anspruch 7, dadurch gekennzeichnet, daß der Haltetrichter (27) aus einem flexiblen, härteren Material als der Ableitschlauch (25) besteht und eine größere, obere Trichtereinlaßöffnung (28) und eine kleinere untere Trichterauslaßöffnung (29) aufweist.

9. Vorrichtung nach den Ansprüchen 7 und 8, dadurch gekennzeichnet, daß der Haltetrichter (27) an seiner größeren Trichteröffnung mit einem konkav geschwungenen oberen Rand (33) versehen ist.

10. Vorrichtung nach den Ansprüchen 7 bis 9, dadurch gekennzeichnet, daß der Haltetrichter (27) an seiner Vorderkante (30) einstückig geschlossen und an seiner Hinterkante (31) mit ihn schließenden Verbindungsmitteln (32, 32a, 32b) versehen ist, wobei die größere, obere Trichtereinlaßöffnung (28) in ihren Abmessungen etwa den Abmessungen des asymmetrischen Trichterbereichs (26) des Urinableiters (10b) und die kleinere untere Trichterauslaßöffnung (29) in ihren Abmessungen etwa den Abmessungen des einstückig angeformten, asymmetrischen Ableitschlauchs (25) des Urinableiters (10b) entspricht.
